# EUROPEAN PATENT SPECIFICATION

(11) **EP 3 337 445 B1**
(45) Date of publication and mention of the grant of the patent: **19.06.2019**
(21) Application number: 16756651.2
(22) Date of filing: 15.08.2016
(51) Int. Cl.: A61K 8/29, A61K 8/81, A61Q 19/02, A61K 8/02

(54) **A COSMETIC MASK**
KOSMETISCHE MASKE
MASQUE COSMÉTIQUE

(30) Priority: 21.08.2015 WO PCT/CN2015/087866; 12.10.2015 EP 15189393
(43) Date of publication of application: 27.06.2018
(73) Proprietor: Unilever NV, 3013 AL Rotterdam (NL); Unilever PLC, London, Greater London EC4Y 0DY (GB)
(72) Inventor: AO, Mingqi, Shanghai 200335 (CN); EKANI NKODO, Axel, Herve, Bebington Wirral Merseyside CH63 3JW (GB); GHATLIA, Naresh, Dhirajlal, Shanghai 200335 (CN); LI, Hangsheng, Shanghai 200335 (CN); YUAN, Caigen, Shanghai 200335 (CN)
(74) Representative: Corsten, Michael Allan
(86) International application number: PCT/EP2016/069332
(87) International publication number: WO 2017/032628

(56) References cited:
- EP-A1- 1 104 670
- WO-A1-02/062312
- US-A1- 2004 018 166

## Description

### Field of the invention

The invention relates to a cosmetic mask. The invention more particularly relates to a cosmetic mask that is a water insoluble substrate with a composition impregnated therein which can be applied to the face or any other topical surface of the body for providing instant lightening benefits without the disadvantages of unnatural whiteness that is often associated with such instant lightening products.

### Background of the invention

Skin lightening is one of the most sought after cosmetic benefits people have been looking for in recent times. The exposure of the skin to the sun and other environmental irritants causes it to darken, often in an uneven and blochy manner. Thus people, not only those living near the tropics who are naturally born with darker skin, but also those who live far from the tropics suffer from such cosmetically unpleasant appearance. There have been many cosmetic approaches used to tackle this problem.

One approach is to use foundations and make-up compositions (also known as colour cosmetics) which are high in coloured dyes or pigments which are applied to the face to mask the unevenness of skin appearance. This approach, although it gives instant change in the skin appearance, are often used more by people in the media and entertainment industry whose images are captured on camera, and transmitted for viewing electronically. For day to day use, people prefer a natural appearance since they are reluctant to appear "heavily made up". Another approach for skin lightening and evenness of skin appearance is to use skin lightening agents which act through biological means to regulate the melanin on the skin thus providing a more lightened appearance. But such biological actives like niacinamide, though effective, take days, if not weeks to deliver visible effectiveness.

Sunscreen compositions comprising organic (UVA and UVB) and/or inorganic sunscreen agents are also widely used. They protect the skin against the natural darkening of the skin when exposed to solar radiation by absorbing and then emitting the UV radiation or by blocking them completely. Inorganic sunscreens like zinc oxide and titanium dioxide are also widely used. These inorganic actives come in various particle sizes. The low particle size (of the order of ten of nanometers) act as sunscreen agents while those in the hundreds of nanometer particle size range are called pigments and offer instant lightening benefits. Unfortunately if higher amounts (higher than say 1% in the composition) is incorporated, they have the disadvantage of un-natural whitening of the skin which is not like by consumers.

The present inventors while being aware of the drawbacks of the above cosmetic technologies took it upon themselves the object of solving the problem to provide for a skin lightening composition which can give instant lightening benefits through incorporation of high amounts of inorganic whitening particles while at the same time giving the skin a natural appearance.

There is another class of cosmetic products which includes products like face mask, clay mask or face packs. Such products consist of a cream or a mud pack or a paper based mask which is applied to the face for a short or long period of time (say a few minutes to several hours e.g. overnight application). In the specific case of a face mask the desired composition is impregnated in a water insoluble substrate e.g. paper and after it is applied for the desired period of time, it is removed. Preferably thereafter the face may be rinsed off with water. Such products have been claimed to give benefits against problems like acne by removing the dead skin cells on the surface and by removing unwanted oil and other irritants. Such products have also been claimed to moisturize the skin through use of high levels of humectants and other moisturizing agents.
Since conventional types of compositions like sunscreens, creams and gels comprising conventional skin lightening agents were not found to give the above benefits, the present inventors explored the as yet unused vehicle of a face mask to study if it can be used to deliver instant lightening benefits. After extensive experimentation they found that it is possible to impregnate specific film forming polymers and high amounts of whitening organic/inorganic particles into a water insoluble substrate and when such a substrate is applied on the skin for a certain period of time and then removed, it is capable of providing the instant lightening benefits. This not only gives enhanced absolute skin lightening scores but the skin was seen to appear more natural

WO02/062132 (Procter and Gamble) discloses a mask composition comprising: (1) a water insoluble substrate; and (2) a liquid composition comprising: (a) a skin tone changing agent selected from the group consisting of skin tone changing pigments, reflective particulate material, and mixtures thereof, wherein the skin tone changing agent has a particle size of at least about 100nm; (b) a water-soluble thickening agent which provides the liquid composition a viscosity of from about 1000 mPa s to about 600,000 mPa.

US6723667 (Kanebo, 2000) discloses a pack comprising a water-soluble polymer and a sazifrage extract. The pack allays irriation and pain on peeling, firm up the skin after use, moisturizes the skin, and has excellent moisturizing properties, quick-drying properties and facility.

A skin whitening mask product is also known from WO 02/062312 (Procter and Gamble). Other masks and pack products are known from US 2004/0018166 and EP 1 104 670.

The above patent publications do not disclose the inventive combination of the present invention i.e. specific film forming polymers and the whitening particles incorporated in a water insoluble substrate for topical application to give the desired benefits.

It is thus an object of the present invention to provide for a mask composition that delivers instant whitening benefits through use of high concentration of organic/inorganic whitening particles while ensuring the retention of even skin appearance in its natural form.

### Summary of the invention

According to the first aspect of the present invention there is provided a cosmetic mask comprising
(i) a water insoluble porous substrate;
(ii) 1 to 15% by weight of film forming polymer; and
(iii) 0.1 to 5% by weight of a whitening particle,
wherein the film forming polymer is water insoluble;
wherein the film forming polymer has a solubility in water at 25 °C of less than 1 wt%; and
wherein the film forming polymer is selected from an acrylate polymer, a methacrylate polymer, a urethane polymer or co-polymers thereof.

According to another aspect of the present invention there is provided a method of lightening skin comprising the steps of
(i) applying the cosmetic mask of the first aspect on to the preferred external skin surface for at least one minute; and
(ii) removing the cosmetic mask from said surface.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are by weight of the entire cosmetic mask including the water insoluble substrate unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

By "A Cosmetic Mask" as used herein, is meant to include a mask for topical application to the skin of mammals, especially humans. The mask, as per this invention, is one that is applied to the desired skin surface and left on for a period of time (say from 1 minute to 12 hours) after which it is removed followed preferably by rinsing the skin surface with water. The mask as per the present invention is primarily intended for skin lighteing purposes but may also be formulated into a product which is applied to a human body for improving the appearance, cleansing, odor control or general aesthetics. "Skin" as used herein is meant to include skin on the face and body (e.g., neck, chest, back, arms, underarms, hands, legs, buttocks and scalp) and especially to the sun exposed parts thereof e.g the face, neck or even parts that are known to darken without sun exposure like the underarm. The mask as per the present invention is especially intended for lightening the face of an individual.

The invention provides for a cosmetic mask comprising a water insoluble porous substrate; a skin lightening composition impregnated in the substrate; wherein the skin lightening composition comprises a film forming polymer; and a whitening particle.

The water insoluble porous substrate for impregnating with the skin lightening composition is selected from paper, polymeric web, fabric, or composites/mixtures thereof. The porous substrate is preferably paper or fabric. The cosmetic mask preferably comprises 1 to 10%, preferably 2 to 5%, more preferably 3 to 4% by weight of the porous substrate. The porous substrate is preferably configured as discrete sheets, a bundle of such sheets, preferably packed together as a bunch for sale to the consumer. The area of each such sheet is preferably from 100 to 1000 cm², preferably 300 to 500 cm², more preferably 350 to 450 cm². The porous substrate in a further preferred aspect is configured in the shape that can be conveniently applied on the face of an individual with holes for the nose, mouth and eyes.

"Film-forming polymer" as used herein refers to a polymer which is capable of forming cohesive and continuous covering over the hair and/or skin when applied to their surface. Contact angle, as used herein, means the angle at which a water/vapor interface meets a solid surface at a temperature of 25°C. Such an angle may be measured with a goniometer or other water droplet shape analysis systems with water droplet of 5 µl and at 25°C. The requirement for film-forming polymer, as per the present invention is that the film-forming polymer is suitable to be employed in cosmetic composition. The film-forming polymer, for use in the present invention, preferably has a contact angle of at least 85°. Not wishing to be bound by theory, the inventors believe that such a compact and continuous film covers the skin surface and helps to reduce the loss of the whitening particles during washing or later through abrasion. The film forming polymer is generally water insoluble and is distinct from water soluble polymers like cross-linked polyacrylic acid sold as Carbopol which are commonly used in cosmetic product as thickeners. The solubility of the polymer in water at 25 °C is less than 1 wt%, preferably less than 0.5 wt%, more preferably less than 0.1 wt% and most preferably less then 0.01 wt%.

The film forming polymer as per the invention is selected from an acrylate polymer, a methacrylate polymer, a urethane polymer or co-polymers thereof. Especially preferred are acrylate co-polymer and a urethane-acrylic co-polymer. Commercially available film forming polymers which may be used in the present invention are KOBO-50N available from Kobo and Hybridur 875 from Air Products. The film forming polymer is included in 1 to 15%, preferably in 1 to 8 %, more preferably in 2 to 6% by weight of the cosmetic mask.

A whitening particle is included in the cosmetic composition for impregnation in the cosmetic mask of the invention. The whitening particle is selected from one of a polymer bead or an inorganic particle with a refractive index higher than 1.8. When a polymer bead is included, it is preferably a hollow sphere. Suitable hollow spheres are those made of a styrene/ acrylate co-polymer, polyurethane, or polyethylene. Polymer beads which may be included in the composition of the invention preferably have a particle size in the range of 80 to 700 nm. Commercially available hollow sphere polymer beads which may be used in the composition of the invention are Sunspheres™ (from Dow).

Alternatively and preferably the whitening particles are inorganic particles with a refractive index higher than 1.8, preferably higher than 2.0, more preferably higher than 2.5. Preferred inorganic particles as per the invention are titanium dioxide, zinc oxide, boron nitride or mixtures thereof. Most preferred inorganic particle is titanium dioxide. The inorganic particles for inclusion in the cosmetic mask of the invention preferably have a mean particle size from 15 nm to 1 micron, preferably 80 to 400 nm. Commercially available titanium dioxide are available as Korons1171 from Korons and KowetTiO 203975 from Sensient . The whitening particle is included in 0.1 to 5%, preferably 0.2 to 4.0%, more preferably 0.2 to 2.0% by weight of the cosmetic mask.

The cosmetic mask of the invention may additionally comprise a surfactant. A surfactant of the anionic or non-ionic form is especially preferred. Suitable surfactants include fatty alcohols and esters of fatty acids. Especially preferred and widely and inexpensively available surfactants of the above classes which may be included in the compositions of the present invention are cetearyl alcohol, glyceryl monostearate and PEG-20 stearate and (PEG-60 stearate). Such surfactants are preferably included in 0.1% to 20%, more preferably included in 0.1-8% by weight of the cosmetic mask.

The cosmetic mask preferably additionally comprises water. Water may be present in 20 to 96%, preferably 40 to 90%, more preferably 50 to 80% by weight of the cosmetic mask.

The cosmetic mask of the present invention can comprise a wide range of other optional components. The CTFA Personal care Ingredient Handbook, Second Edition, 1992, which is incorporated by reference herein in its entirety, describes a wide variety of non-limiting personal care and pharmaceutical ingredients commonly used in the skin care industry, which are suitable for use in the compositions of the present invention. Examples include: antioxidants, binders, biological additives, buffering agents, colorants, thickeners, polymers, astringents, fragrance, opacifying agents, conditioners, exfoliating agents, pH adjusters, preservatives, natural extracts, essential oils, skin sensates, skin soothing agents, and skin healing agents.

According to another aspect of the present invention there is provided a method of lightening skin comprising the steps of applying the cosmetic mask of the invention on to the preferred external skin surface for at least one minute and removing the mask from the surface. Preferably thereafter the surface is rinsed with water. The method is preferably non-therapeutic. The mask is preferably left on to the desired skin surface from 1 minute to 12 hours, preferably 5 minutes to 4 hours, more preferably 10 minutes to an hour before it may be removed. The desired skin surface is rinsed with water from 1 minute to 2 hours, preferably 5 minutes to an hour after removing the cosmetic mask.

The invention will now be illustrated with the help of the following non-limiting examples.

### Examples

### Examples 1 to 4: Effect of inclusion of TiO₂ and film forming polymer in a face mask

The cosmetic masks were prepared as described below.

Water insoluble porous substrate (non-woven fabric in this case) was immersed into 20 ml of an emulsion and the mask was sealed in an individual package.

The masks were impregnated with the following compositions as shown in Table -1 below. The cosmetic masks were evaluated for its efficacy in providing skin lightening to skin. The procedure used was to apply the mask, wash the surface and measure the efficacy as given below:

### Protocol 1:

The non-woven fabric with the impregnated emulsion was spread on a 50#Bio-Skin Plate (50#BSP) for 15 minutes. Then the fabric was peeled off, and the 50#BSP was rinsed by tap water for 15 seconds. After 50#BSP was completely dry, L* value were measured. ΔL was obtained from (Lₛₐₘₚₗₑ - L_{blank}). Lₛₐₘₚₗₑ is the L values of 50#BSP with mask after wash, L_{blank} is the L value of blank 50#BSP.

The skin lightening efficacy is measured using the above protocol and the ΔL values for the various samples are summarized in Table -1.

**Table -1**

| Ingredients | Example1 | Example 2 | Example 3 | Example 4 |
|---|---|---|---|---|
| Fabric, wt% | 3.64 | 3.64 | 3.64 | 3.64 |
| Kowet, TiO₂, wt% | 0.24 | 0.24 | 0.24 | 0.48 |
| Kobo 50N, wt% | - | 3.85 | 5.78 | 3.85 |
| Water, wt% | To 100 | To 100 | To 100 | To 100 |
| ΔL | 0.34 | 0.45 | 0.87 | 0.93 |

Kowet TiO₂ is: is a kind of hydrophilic TiO₂ particle with size from 500 nm to 2 µm.

Kobo 50N is: is a formulation containing 48.5 to 51.5% film forming polymer of acrylate type, 1.3 to 1.7% laureth-21 non-ionic surfactant in water.

### Example 5-8: Samples similar to Examples 1-4 except that a different washing protocol was used

Samples as per Example 1 -4 were taken and applied on to the desired surface but the washing procedure was modified as given below:

### Protocol 2:

The non-woven fabric with emulsion was spread on a 50#Bio-Skin Plate (50#BSP) for 15 minutes. Then the fabric was peeled off, and the 50#BSP was dried for 1 hour at room temperature (25 °C). After that, 50#BSP was rinsed with tap water for 15 seconds, and the L* value was measured after it was completely dry. ΔL was obtained from (Lₛₐₘₚₗₑ - L_{blank)}. Lₛₐₘₚₗₑ is the L values of 50#BSP with mask after wash, L_{blank} is the L value of blank 50#BSP.

The skin lightening data is summarized in Table - 2 below:

**Table -2**

| Ingredients | Example-5 | Example-6 | Example-7 | Example-8 |
|---|---|---|---|---|
| ΔL | 1.49 | 1.78 | 2.30 | 6.20 |

The data in Table - 1 and 2 indicates that when a combination of a whitening particle (titanium dioxide) and a film forming polymer are incorporated in a water insoluble substrate and applied on skin, one is able to get high skin lightening efficacy using very different washing protocols.

### Examples 9-13: Effect of using another whitening particle (hollow polymeric microspheres)

Cosmetic masks having the ingredients as shown in Table - 3 were prepared and applied similar to the earlier examples. The face was washed using Protocol 1 and the skin lightening efficacy (measured as ΔL) is summarized in Table -3.

**Table - 3**

| Ingredients | Example9 | Example 10 | Example 11 | Example 12 | Example 13 |
|---|---|---|---|---|---|
| Fabric, wt% | 3.64 | 3.64 | 3.64 | 3.64 | 3.64 |
| Sunspheres, wt% | 1.93 | 1.93 | 1.93 | 1.93 | 1.93 |
| Kobo 50N, wt% | - | 1.93 | 3.85 | 5.78 | 7.71 |
| Water,wt% | To 100 | To 100 | To 100 | To 100 | To 100 |
| ΔL | 0.27 | 0.87 | 1.02 | 1.32 | 1.30 |

Sunspheres: is a hollow microsphere polymer made of styrene/ acrylate co-polymer with an average particle size of 400nm sourced from Dow Chemicals.

The samples as per Table - 3 were subjected to Protocol 2 and the ΔL values are summarized in Table - 4.

**Table - 4**

| Ingredients | Example14 | Example 15 | Example 16 | Example 17 | Example 18 |
|---|---|---|---|---|---|
| ΔL | 2.64 | 4.57 | 4.90 | 5.31 | 5.39 |

The data in Table - 3 and 4 indicates that when a combination of a whitening particle (hollow polymeric microsphere) and a film forming polymer are incorporated in a water insoluble substrate and applied on skin, one is able to get high skin lightening efficacy using very different washing protocols.

### Examples 19-20: Effect of using different amounts of hollow microspheres

Samples as shown in Table -5 were prepared with different amounts of hollow polymeric microspheres and the skin lightening efficacy measured after the skin was subjected to Protocol 2 and the data is summarized therein.

**Table-5**

| Ingredients | Example19 | Example 16 | Example 20 |
|---|---|---|---|
| Fabric, wt% | 3.64 | 3.64 | 3,64 |
| Sunsphere, wt% | 0.96 | 1.93 | 3.85 |
| Kobo 50N, wt% | 3.85 | 3.85 | 3.85 |
| Water,wt% | To 100 | To 100 | To 100 |
| ΔL | 2.17 | 4.90 | 8.94 |

The data in Table-5 indicates that various amounts of hollow polymeric microspheres can be used in combination with a film forming polymer in a cosmetic mask to get good skin lightening efficacy.

### Examples 21-24: Effect of different types of whitening particles and film forming polymers

Samples as shown in Table -6 were prepared and the skin lightening efficacy measured after subjecting the skin to Protocol 2.

**Table -6**

| Ingredients | Example 21 | Example 22 | Example 23 | Example 24 |
|---|---|---|---|---|
| Fabric, wt% | 3.64 | 3.64 | 3.64 | 3.64 |
| Particle, type | Boron Nitride | Kowet, TiO₂ | Sunsphere | Boron Nitride |
| Particle, wt% | 0.48 | 0.48 | 1.93 | 0.48 |
| Polymer, type | Kobo 50N | H875 | H875 | H875 |
| Polymer, wt% | 3.85 | 4.82 | 4.82 | 4.82 |
| Water, wt% | To 100 | To 100 | To 100 | To 100 |
| ΔL | 3.46 | 6.77 | 3.52 | 2.26 |

Boron Nitride: was chemical pure grade from Momentive.
H875 is Polyurethane-2 and Polymethyl Methacrylate co-polymer from Air Products.

The data in Table -6 indicates that various different types of whitening particles in combination with different film forming polymers can be incorporated in a cosmetic mask for getting excellent skin lightening benefits.

## Claims

1. A cosmetic mask comprising
(i) a water insoluble porous substrate;
(ii) 1 to 15% by weight of film forming polymer; and
(iii) 0.1 to 5% by weight of a whitening particle,
wherein the film forming polymer is water insoluble; wherein the film forming polymer has a solubility in water at 25 °C of less than 1 wt%; and
wherein the film forming polymer is selected from an acrylate polymer, a methacrylate polymer, a urethane polymer or co-polymers thereof.

2. A cosmetic mask as claimed in claim 1 wherein said water insoluble porous substrate is selected from paper, polymeric web, fabric, or composites/mixtures thereof.

3. A cosmetic mask as claimed in claim 2 wherein said polymer is an acrylate copolymer or a polyurethane-methylmethacrylate co-polymer.

4. A cosmetic mask as claimed in any one of the preceding claims wherein said whitening particle is selected from a polymer bead or an inorganic particle with a refractive index higher than 1.8.

5. A cosmetic mask as claimed in claim 4 wherein said whitening particle is a hollow sphere made of a styrene/ acrylate co-polymer, titanium dioxide, zinc oxide, boron nitride or mixtures thereof.

6. A cosmetic mask as claimed in any one of the preceding claims wherein the whitening particle has a particle size from 100 nm to 10 microns.

7. A cosmetic mask as claimed in any one of the preceding claims wherein additionally comprising 40 to 96% water.

8. A cosmetic method of lightening skin comprising the steps of
(i) applying the cosmetic mask as claimed in any one of the preceding claims on to the preferred external skin surface for at least one minute; and
(ii) removing the cosmetic mask from said surface.

9. A method as claimed in claim 8 wherein the surface is rinsed with water after step (ii).

10. A method as claimed in claim 8 or 9 wherein said step (ii) is carried out from 0 to 60 minutes after step (i).

## Patentansprüche

1. Kosmetische Maske, umfassend
(i) ein wasserunlösliches poröses Substrat;
(ii) 1 bis 15 Gewichts-% filmbildendes Polymer; und
(iii) 0,1 bis 5 Gewichts-% eines Aufhellerteilchens,
wobei das filmbildende Polymer wasserunlöslich ist;
wobei das filmbildende Polymer eine Löslichkeit in Wasser bei 25°C von weniger als 1 Gew.-% aufweist; und
wobei das filmbildende Polymer unter einem Acrylat-Polymer, einem Methacrylat-Polymer, einem Urethan-Polymer oder Copolymeren davon ausgewählt ist.

2. Kosmetische Maske, wie im Anspruch 1 beansprucht, wobei das wasserunlösliche poröse Substrat unter Papier, Polymergewebe, Textil oder Verbundstoffen/Mischungen davon ausgewählt ist.

3. Kosmetische Maske, wie im Anspruch 2 beansprucht, wobei das Polymer ein Acrylat-Copolymer oder ein Polyurethan-Methylmethacrylat-Copolymer darstellt.

4. Kosmetische Maske, wie in irgendeinem der vorhergehenden Ansprüche beansprucht, wobei das Aufhellerteilchen aus Polymerkügelchen oder einem anorganischen Teilchen mit einem Brechungsindex von höher als 1,8 ausgewählt ist.

5. Kosmetische Maske, wie im Anspruch 4 beansprucht, wobei das Aufhellerteilchen eine Hohlkugel darstellt, die aus einem Styrol/Acrylat-Copolymer, Titandioxid, Zinkoxid, Bornitrid oder Mischungen davon hergestellt ist.

6. Kosmetische Maske, wie in irgendeinem der vorhergehenden Ansprüche beansprucht, wobei das Aufhellerteilchen eine Teilchengröße von 100 nm bis 10 Mikrometer aufweist.

7. Kosmetische Maske, wie in irgendeinem der vorhergehenden Ansprüche beansprucht, wobei diese zusätzlich 40 bis 96% Wasser umfasst.

8. Kosmetisches Verfahren zur Hautaufhellung, umfassend die Schritte
(i) Auftragen der kosmetischen Maske, wie in irgendeinem der vorhergehenden Ansprüche beansprucht, auf die bevorzugte äußere Hautoberfläche für mindestens eine Minute; und
(ii) Entfernen der kosmetischen Maske von der Oberfläche.

9. Verfahren, wie im Anspruch 8 beansprucht, wobei die Oberfläche nach Schritt mit Wasser (ii) abgespült wird.

10. Verfahren, wie im Anspruch 8 oder 9 beansprucht, wobei der Schritt (ii) 0 bis 60 Minuten nach Schritt (i) durchgeführt wird.

## Revendications

1. Masque cométique comprenant
(i) un substrat poreux insoluble dans l'eau ;
(ii) de 1 à 15 % en masse de polymère formant un film ; et
(iii) de 0,1 à 5 % en masse d'une particule blanchissante,
dans lequel le polymère formant un film est insoluble dans l'eau ;
dans lequel le polymère formant un film présente une solubilité dans l'eau à 25°C inférieure à 1 % en masse ; et
dans lequel le polymère formant un film est choisi parmi un polymère d'acrylate, un polymère de méthacrylate, un polymère d'uréthane ou des copolymères de ceux-ci.

2. Masque cosmétique selon la revendication 1, dans lequel ledit substrat poreux insoluble dans l'eau est choisi parmi du papier, une bande polymère, un tissu, ou des composites/mélanges de ceux-ci.

3. Masque cosmétique selon la revendication 2, dans lequel ledit polymère est un copolymère d'acrylate ou un copolymère de polyuréthane-méthacrylate de méthyle.

4. Masque cosmétique selon l'une quelconque des revendications précédentes, dans lequel ladite particule blanchissante est choisie parmi une bille polymère ou une particule inorganique avec un indice de réfraction supérieur à 1,8.

5. Masque cosmétique selon la revendication 4, dans lequel ladite particule blanchissante est une sphère creuse constituée d'un copolymère de styrène/acrylate, dioxyde de titane, oxyde de zinc, nitrure de bore ou de mélanges de ceux-ci.

6. Masque cosmétique selon l'une quelconque des revendications précédentes, dans lequel la particule blanchissante présente une taille de particule de 100 nm à 10 microns.

7. Masque cosmétique selon l'une quelconque des revendications précédentes, comprenant de plus de 40 à 96 % d'eau.

8. Procédé cosmétique d'éclaircissement de la peau comprenant les étapes de
(i) application du masque cosmétique selon l'une quelconque des revendications précédentes sur la surface de peau externe préférée pendant au moins une minute ; et
(ii) élimination du masque cosmétique de ladite surface

9. Procédé selon la revendication 8, dans lequel la surface est rincée avec de l'eau après l'étape (ii).

10. Procédé selon la revendication 8 ou 9, dans lequel ladite étape (ii) est réalisée de 0 à 60 minutes après l'étape (i).
